# EUROPEAN PATENT SPECIFICATION

(11) **EP 4 365 575 B1**
(45) Date of publication and mention of the grant of the patent: **02.07.2025**
(21) Application number: 22205044.5
(22) Date of filing: 02.11.2022
(51) Int. Cl.: G01N 21/17, A61B 5/00, A61B 8/00, G01N 21/27, A61B 5/145, G01N 29/24

(54) **PHOTOACOUSTIC DETECTING DEVICE COMPRISING A HOUSING KIT**
VORRICHTUNG ZUR PHOTOAKUSTISCHEN DETEKTION UMFASSEND EINEN GEHÄUSEKIT
DISPOSITIF DE DÉTECTION PHOTOACOUSTIQUE COMPRENANT UN KIT DE BOÎTIER

(43) Date of publication of application: 08.05.2024
(73) Proprietor: Eclypia, 38000 Grenoble (FR)
(72) Inventor: BERNET, Florent, 38100 Grenoble (FR); COUTARD, Jean-Guillaume, 38660 Saint Pancrasse (FR); BOUCHER-DOIGNEAU, Morgane, 38000 Grenoble (FR)
(74) Representative: Fidal Innovation

(56) References cited:
- WO-A1-2012/117719
- AU-A1- 2014 213 497
- US-A1- 2009 216 121
- US-A1- 2014 073 899
- US-A1- 2014 114 170
- US-A1- 2017 164 839
- US-A1- 2020 069 189
- US-A1- 2021 302 387

## Description

### FIELD OF THE INVENTION

The present invention relates to a photoacoustic detecting device for measuring a parameter of interest in a medium.

### BACKGROUND OF THE INVENTION

Photoacoustic detection may be used in the field of detecting devices, notably for detecting parameters of interest such as chemical components in a medium. The medium may be an organic tissue, such as the skin of a human.

The photoacoustic detection is based on the irradiation of a medium M to be analysed by a light signal emitted by a light source. Generally, the light source is one or several lasers, in particular a quantum cascade laser (QCL). The light signal is a light beam of a chosen wavelength. The wavelength is chosen regarding the type of parameter of interest to be measured.

The photoacoustic detection is based on the detection of a pressure wave associated with a thermic wave. The thermic wave is generated under the effect of the absorption of the light beam by the medium. Such absorption creates a local heating of chemical components in the medium, where the light beam has been absorbed. The thermic wave propagates in the medium M before propagating outside the medium. More precisely, when the thermic wave comes out of the medium, after its propagation, a pressure wave is generated, which can be detected.

The photoacoustic detection may be made specific to particular chemical components, by adjusting the wavelength and/or the modulation frequency of the light beam. More precisely, the wavelength may be adjusted to match an absorption peak of the component to analyse.

Photoacoustic detection then is a non-invasive way of analysing a medium of interest. Numerous photoacoustic detecting devices have been developed.

Generally, such photoacoustic detecting devices are intended to be constantly worn by a person, for example on the arm of a person. The medium to be analysed is typically the skin of the person and a parameter of interest is a chemical component present in the skin of the person. Advantageously, the detecting device is worn constantly by the person and is monitoring continuously the parameter of interest of the person.

The device comprises a photoacoustic cell comprising a contact face, intended to be applied, in contact, with the skin of the person wearing the device. A light signal is emitted by a light source and guided by a guide through the photoacoustic cell to reach the skin of the person. Generally, the contact surface of the photoacoustic cell presents an aperture through which the light signal reaches the skin. A photoacoustic transducer is provided to detect a photoacoustic wave extending through the cavity, which is then analysed to detect a parameter of interest. Such photoacoustic detecting device is described in document EP3885765.

Such photoacoustic device presents two main challenges. The first challenge is that the contact face of the photoacoustic cell must stay in contact with the medium to be analysed when the detection is made and that the photoacoustic device must be regularly calibrated, to avoid any measure artefact

To answer this first challenge, a photoacoustic detecting device may be provided with a photoacoustic cell able to move inside the housing and with elastic element configured to compensate the movement of the photoacoustic cell, such that the contact surface of the photoacoustic cell remains in constant contact with the medium to analyse.

Regarding the second challenge, when the photoacoustic cell is able to move in the housing, it is difficult to easily calibrate the detecting device when some movements of the building block occur. It follows that for such devices, either the movement of the building block is not considered during the calibration, leading to a poor result, or the movement of the building block must be known exactly to be compensated during the calibration, which is difficult to perform and cannot be done by the person wearing the detecting device.

Hence, when the person needs to calibrate the detecting device, they would have to send back the detecting device to the fabricant which is time consuming. Moreover, to continuously perform the analysis of the parameter of interest of the person, the person would need several detecting devices to not wait for the return of their calibrated detecting device, which is costly.

### BRIEF SUMMARY OF THE INVENTION

The present application answers at least partially the above exposed technical problem.

To this aim, the invention relates to a photoacoustic detecting device for measuring a parameter of interest in a medium comprising:
➢ a housing kit comprising:
   ➢ a calibrating cover,
   ➢ a functioning cover,
   ➢ a baseplate,
   ➢ the calibrating cover and the functioning cover being interchangeable with one another, such that,
      o when the baseplate is attached to the functioning cover, the baseplate and the functioning cover are configured to form a functioning housing allowing an analysis of the medium by the photoacoustic detecting device,
      o when the baseplate is attached to the calibrating cover, the baseplate and the calibrating cover are configured to form a calibrating housing allowing a calibration of the photoacoustic detecting device,
   ➢ a building block freely housed in the housing, said housing being either the functioning housing or the calibrating housing, said building block comprising:
      o a light source, configured to emit a light signal,
      o a photoacoustic cell comprising a contact surface with an opening intended to be in contact with the medium, whereby the light signal is able to propagate in the photoacoustic cell and pass through the opening in the contact surface to reach the medium, the contact surface of the photoacoustic cell being configured to emerge out of an aperture of the baseplate,
      o a guiding element, configured to direct the light signal toward the photoacoustic cell,
      o a transducer assembled to the photoacoustic cell and configured to detect a generated signal, where the generated signal is generated in the photoacoustic cell by a photothermic effect in the medium in response to an irradiation of the medium by the light signal,
      o one or more maintaining elements configured to assemble the light source, the transducer, the photoacoustic cell and said guide in a single building block,
wherein
➢ in the functioning housing, elastic elements are provided between the functioning cover and the building block, the functioning cover being configured to allow a movement of the building block inside the housing, said elastic element being configured to elastically deform under the effect of the movement of the building block relatively to the housing in order to compensate said movement of said building block,
➢ the calibrating cover comprises blocking elements, said blocking elements being configured to block any movement of the building block relatively to the housing.

Such photoacoustic devices are intended to be worn as much as possible by a person. The providing of a photoacoustic device with two covers allows to answer to the two main challenges being the remaining of the hollow contact surface against the medium to analysed and the calibration of such detecting device.

First, the configuration of the functioning housing allows to efficiently compensate for movement of the single building block relatively to the housing, therefore allowing accurate measurement of the parameter of interest by avoiding any artefact measure. Indeed, since the photoacoustic cell emerges out of an aperture of the baseplate, a movement of the medium against the photoacoustic cell may cause the photoacoustic cell to displace, for example such movement may push the photoacoustic cell inside the housing where the detection can no longer be made with accuracy. The elastic components allow to compensate for such displacement of the photoacoustic cell, such that the photoacoustic cell is able to stay in contact with the medium at any time.

Second, the configuration of the calibrating housing allows to avoid any movement of the building block that would need to be compensated during the calibration, which is time and resource consuming.

The provision of two covers allows a great time saving and a diminution of calculating resources regarding the calibration, while allowing accurate analysis of the medium, thanks to the functioning cover.

Furthermore, another advantage of providing a detecting device with two covers occurs during the fabrication process of the detecting device. More precisely, after the assembly of the building block inside the baseplate, a calibrating cover may be used on all devices to verify that the movement of the building block inside the housing is blocked, thereby ensuring that all the components have been well placed. A single calibrating cover may be used for all the devices being fabricated such that the fabricant will be sure that all the devices have the same mechanical structure and the same placement of the components inside the housing. Hence, the use of a calibrating cover during the fabrication process of the devices allows to ensure the conformity of all the detecting devices. Also, when the calibrating cover is placed on the products, a photoacoustic measurement can be done on all the devices using said same calibrating cover. Following the photoacoustic measurement, the devices may be calibrated such that all the devices will be calibrated according to the same configuration. Then, the fabrication process of the devices may continue until they are sent to the users. This allows having controlled, identical and maintainable fabrication and calibration processes for all the devices.

According to different aspects, it is possible to provide the one and / or the other of the characteristics below taken alone or in combination.

According to one embodiment, when the building block is housed in the functioning housing, the building block and an inner upper surface of the functioning cover are spaced from one another, the elastic element being provided in said space, wherein the building block is configured to move in said space according to an upward-downward movement relatively to the functioning housing and wherein the elastic element is configured to elastically deform between the functioning cover and the building block according to the upward-downward movement of said building block in the functioning housing.

Such configuration of the functioning cover allows the elastic elements to absorb the energy generated by the displacement of the building block toward the top of the functioning housing by deforming elastically. The movement of the building block therefore can be compensated, allowing the hollow contact surface of the cell to stay in contact with the medium.

According to one embodiment, an upper interior surface of the functioning cover further comprises a cooperating element cooperating with said elastic element.

The cooperating elements may be plots positioned with regards to the elastic elements. Such plots may allo to use a smaller elastic element.

According to an embodiment, the elastic element is provided on the inner upper interior surface of the functioning cover.

According to an embodiment, the elastic element is provided on the building block.

More precisely, the elastic element may be provided on a face of the maintaining element attaching the components of the building block, where said face faces the inner upper surface of the cover. Such maintaining element may be a PCB.

According to an embodiment, the elastic element is a spring leaf.

The attachment of the elastic elements directly to the maintaining element allows an easy fabrication since the machinery used to construct the maintaining element can be used to install the elastic elements on the maintaining element. The installation can then be made in one single step. Furthermore, no fixing elements such as glue or screws need to be used to fix the elastic element to the maintaining element. This is especially the case when the elastic elements are leaf springs and the maintaining element a PCB.

According to an embodiment, when the building block is housed in the calibrating housing, the building block and an inner upper surface of the calibrating cover are spaced from one another, the blocking element of said calibrating cover comprising a plot of a length equal to said space, said plot thereby being able to maintain the building block against the baseplate.

Thus, the plots allow to block any movement of the building block inside the calibrating cover. Furthermore, the plots and the calibrating cover may be formed as a single piece, in a single fabrication step, then reducing the cost and facilitating the fabrication.

According to an embodiment, the blocking element of the calibrating cover comprises a height of said calibrating cover wherein said height of said calibrating cover is such that a height of the calibrating housing is equal to a height of the building block, thereby pressing the building block between said calibrating cover and said baseplate.

In this configuration, there is no need for any further blocking element since the measures of the calibrating cover are chosen to block any movement of the building block inside the calibrating housing.

According to an embodiment, the baseplate comprises blocking elements on its lateral inner surface, said blocking elements being configured to block any lateral movement of the building block inside the housing.

Thus, the building block cannot move laterally in the functioning housing and in the calibrating housing.

### BRIEF DESCRIPTION OF THE DRAWINGS

Embodiments of the invention will be described below with reference to the drawings, described briefly below:
[Fig. 1] represents a detecting device worn by a person.
[Fig. 2] represents a block schema of a photoacoustic detecting device.
[Fig. 3] represents a detecting device in a functioning housing according to an embodiment.
[Fig. 4] illustrates a detecting device in a functioning housing according to another embodiment.
[Fig. 5] illustrates an example of lateral blocking elements of a detecting device in a functioning housing.
[Fig. 6] represents a detecting device in a calibrating housing according to an embodiment.
[Fig. 7] illustrates an example of lateral blocking elements of a detecting device in a calibrating housing.
In the drawings, identical references designate identical or similar objects.

### DETAILED DESCRIPTION OF THE INVENTION

The invention relates to a photoacoustic detecting device 1 for measuring a parameter of interest in a medium M.

In a non-limiting example, the photoacoustic detecting device 1 (or "detecting device 1" in the following description) is intended to be worn by a person P. The medium M may be an organic tissue such as the skin of the person P wearing the detecting device 1.

A parameter of interest may be a chemical component present in the skin of the person P, such as molecules. The parameter of interest may comprise glucose, cholesterol, triglyceride, urea, albumin, and/or alcohol. This list is non exhaustive and several other parameters of interest may be measured.

The measured parameters may then be analysed to determine a blood concentration of glucose, cholesterol and so on.

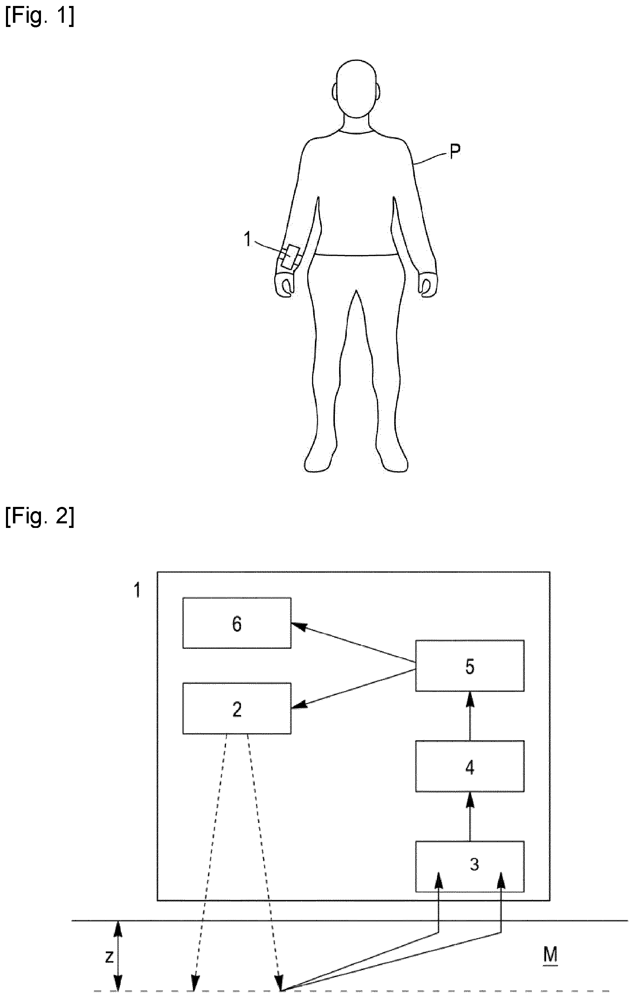

As seen on figure 1, the detecting device 1 may be worn on the arm, or wrist, of a person P. The detecting device 1 may be fixed to the arm of the person P by means of a bracelet.

The detecting device 1 may allow a continuous monitoring of the person P, by repeatedly measuring the parameters of interest of the person P, while it is worn.

Figure 2 is a block diagram of the detecting device 1.

The detecting device 1 comprises:
at least one light source 2, configured to emit a light signal.
a transducer 3 acquiring a signal coming from the medium M,
a signal processing module 4 for analysing the signal detected by the transducer.

In an embodiment, the transducer 3 may be an acoustic transducer 3 detecting an acoustic signal generated in response to the irradiation of the medium M by the light signal

The transducer 3 can be connected to the signal processing module 4 such that the signal processing module 4 receives a signal coming from the transducer 3.

In a non-limiting embodiment, the signal processing module 4 may comprise an analogic-to-numeric converter, converting the signal acquired by the transducer 3 to a numeric signal

In another embodiment, the transducer 3 may directly transmit a numeric signal to the signal processing module 4.

The signal processing module 4 may be implemented in a processor (not shown) which may or not be remote from the detecting device 1.

The detecting device 1 may comprise other components. For example, the detecting device 1 may also comprise an adapting module 5 for adapting irradiation parameters of the light source 2 and a memory 6, for example. These components won't be further described.

In an embodiment, the light source 2 emits a light signal at a chosen wavelength, towards the medium M to be analysed. The chosen wavelength may be chosen according to the parameters of interest to be measured.

More precisely, the wavelength may correspond to the absorption peak of the parameter of interest to be measured. As an example, to detect glucose, the wavelength may be 1034 cm⁻¹, which corresponds to the absorption peak of the glucose.

The light source may be a laser, and more particularly a quantum cascade laser (QCL). In a variant embodiment, the light source 2 may be an electroluminescent diode (led).

In an embodiment, the detecting device 1 may comprise a plurality of light sources 2. In this embodiment, each light source 2 may emit a light signal at different wavelength. In variant, some light sources 2 emit a light signal at the same wavelength, while other light sources 2 emit a light signal at a different wavelength.

In the following description, the detecting device 1 is described as comprising only one light source 2, while it is understood that the same specification applies for a plurality of light sources 2.

The light signal emitted by the light source 2 propagates to the medium M and through it. This phenomenon is represented by the dotted arrows on figure 2. The light signal is absorbed by the constituents of the medium M under a depth z depending on the chosen wavelength of the light signal, the frequency of the light signal and of the composition of the medium M.

The absorption of the light signal energy causes a local heating of the medium M. As a consequence, a thermic signal propagates in the medium M (phenomenon illustrated by the full arrows on figure 2), in particular towards the surface of the medium M. Moreover, the thermic wave may create, outside the medium M, a pressure wave that propagates outside the medium M. Such a pressure wave may be detected by the acoustic transducer 3.

In an embodiment, the thermic wave may also be detected by means of a thermic transducer, such as a thermometer, not shown in the figures.

The detecting device 1 may comprise an acoustic and/or a thermic transducer 3. Other types of transducer 3 may be used.

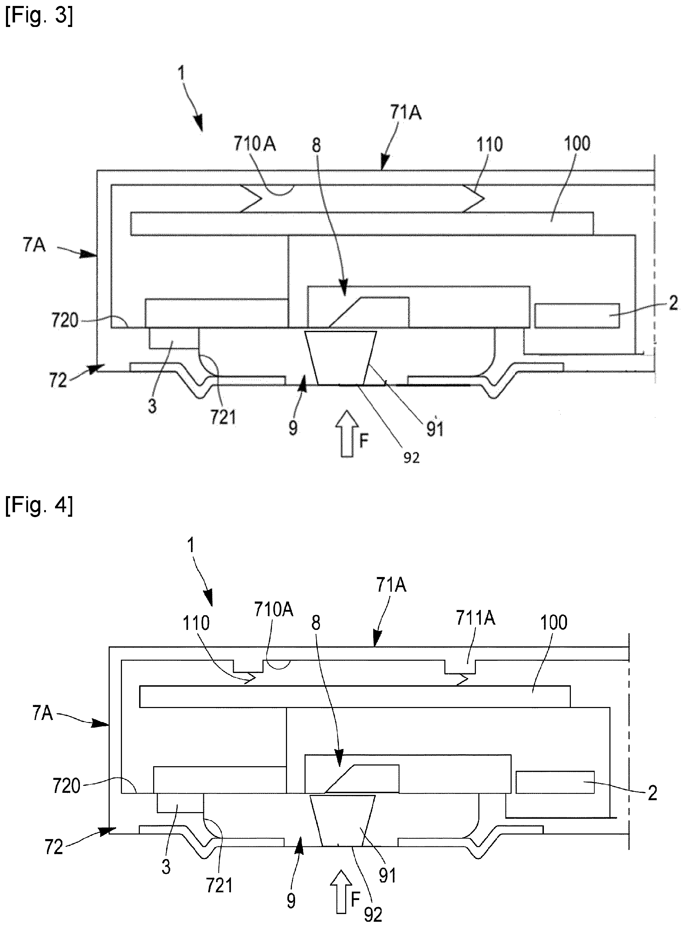

Figure 3 schematically illustrates a detecting device 1 according to an embodiment.

The detecting device 1 comprises a housing 7A, 7B, formed by a cover 71A, 71B and a baseplate 72. The housing 7 comprises an interior delimited by an inner surface 710A, 710B of the cover 71A, 71B and an inner surface 720 of the baseplate 72.

Advantageously, the inner surface of the cover 71A, 71B comprises an upper surface and lateral surfaces and the inner surface of the baseplate 72 comprises a lower surface and lateral surfaces, where the lateral surfaces of the baseplate and of the cover 71A, 7B join each other on their edge in the housing and where the upper surface of the cover and the lower surface of the baseplate face each other in the housing.

The housing 7A, 7B will be described in more details below.

The interior of the housing 7A, 7B, delimited by the inner surface 710A, 710B of the cover 71A, 71B and the inner surface 720 of the baseplate 72, houses a light source 2, which, as described above, may be a QCL.

A guiding element 8 is also provided. The guiding element 8 is configured to direct the light signal emitted by the light source 2 towards the medium M to be analysed.

In the embodiment in which the detecting device 1 comprises a plurality of light sources 2, the guiding element 8 may also be configured to collimate each light signal emitted by each light source 2 into a single light signal directed towards the medium M to be analysed.

More particularly, the guiding element 8 is configured to direct the light signal emitted by the light source 2 through a photoacoustic cell 9 (or "cell 9" in the following description), placed between the medium M to be analysed and the light source 2.

The cell 9 comprises a cavity 91 extending from from the guiding element 8 to a hollow contact surface 92 of said cell 9 through which the medium M is accessible. The light signal emitted by the light source 2 propagates in the photoacoustic cell 9 by first entering the cavity 91 and then passing through the hollow contact surface 92.

The hollow contact surface 92 may have the shape of a circle and the hollow may be positioned on the centre of the circle and may present also a general circular shape. Other configurations are possible.

To close the cavity 91, a window of transparent material may be placed on the hollow part of the hollow contact surface 92. The window may be made of transparent material allowing the light signal to traverse it, for example silicon.

The hollow surface contact 92 may be intended to be placed against the medium M to be analysed, and more precisely may be placed in direct contact with the medium M to be analysed.

Then, when the detecting device 1 is worn by a person P, as represented on figure 1, the hollow contact surface 92 is directly placed against the skin of the person P.

To this end, the base plate 72 comprises an aperture 721 from which the hollow contact surface 92 emerges. Thus, the hollow contact surface 92 of the cell 9 is positioned outside the interior of the housing 7 and can be placed in direct contact with the medium M to analyse.

Regarding the photoacoustic detection, a thermic wave is generated under the effect of the absorption by the medium of the light beam emitted by the light source. Such absorption creates a local heating of chemical components in the medium, where the light beam has been absorbed. The thermic wave propagates in the medium M before propagating outside the medium. When the thermic wave comes out of the medium, after its propagation, a pressure wave is generated, which propagates in the cavity 91 of the cell 9 where it can be detected. The pressure wave, i.e. an acoustic wave, is then induced by the photothermic effect.

The transducer 3 is located in the interior of the housing 7A, 7B and is preferably assembled to the cell 9, such that the transducer 3 can detect and measure the generated pressure wave.

Furthermore, the detecting device 1 comprises one or several maintaining elements. Such maintaining element 100 may be a metallic plate, a plastic plate etc. The maintaining element is used to maintain at least the light source 2, the transducer 3, the guiding element 8 and the cell 9 together. More precisely, the light source 2, the transducer 3, the guiding element 8 and the cell 9 are attached together via one or more maintaining element, to form one single building block. The single building block is considered kinematically as a rigid body in normal use.

In a preferred embodiment, the maintaining element 100 is one or more printed circuit board(s) (PCB) 100. In the following description, it is considered that the maintaining element is a PCB 100, while other applications and embodiments enter within the scope of the present application.

The PCB 100 also ensures the electronic and/or electric connections between the components of the building block.

The building block is freely received in the housing 7A, 7B. By "freely received", it means that the building block is neither attached to the baseplate 72, nor to the cover 71A, 71B. In other words, there is no fixing element, such as screw, glue etc., that fixes the building block to the housing 7A, 7B.

In the embodiment represented on figure 3, the building block comprises, from bottom to top, where the bottom is situated near the baseplate 72 and the top near the cover 71A, 71B, the cell 9, where the hollow surface contact 92 of the cell 9 emerges out of the baseplate 72 through the aperture 721, the transducer 3, the guiding element 8, the light source 2 and a PCB 100 provided at the top.

Other PCBs or other types of maintaining elements may be provided.

As stated above, to allow an accurate detection of the parameter of interest, and to avoid any measure artefacts, the hollow contact surface 92 of the cell 9 must remain in contact with the medium M to analyse at each time. Moreover, the detecting device 1 has to be regularly calibrated. It is an aim of the present application to provide a detecting device 1 allowing the hollow contact surface 92 of the cell to be in constant contact with the medium to be analysed while allowing an easy calibration of the detecting device 1.

It can be particularly difficult to ensure that the contact surface 92 of the cell remains in contact with the medium when the detecting device 1 is intended to be worn on the arm of a person P, as illustrated on figure 1. Indeed, an arm is not static and any movement of the user could cause a displacement of the detecting device 1, leading to a loss of contact between the hollow contact surface 92 and the skin of the person P. Moreover, the texture itself of the skin could cause some loss of contact, since the skin is, per nature, soft and elastic.

It is also important to provide continuous contact between the medium and the contact surface 92 of the cell 9 since the underlying skin mapping is an important parameter for the accuracy of the measurement. If the device moves, the underlying skin may have a different architecture, and therefore new measurements may not be comparable with old measurements.

Moreover, such detecting device 1 needs to be calibrated regularly to avoid any measure artefact and to provide accurate results.

To solve these technical problems, the detecting device 1 is provided with a housing kit. The housing kit comprises the baseplate 72, a functioning cover 71A and a calibrating cover 71B. The housing 7A, 7B is formed by attaching together the baseplate 72 to the functioning cover 71A or by attaching together the baseplate 72 to the calibrating cover 71B.

More precisely, the functioning cover 71A and the calibrating cover 71B can be reversibly attached to the baseplate 72 when needed and are interchangeable with one another as many times as necessary.

When the functioning cover 71A is attached to the baseplate 72, they form together a functioning housing 7A. The functioning housing 7A is used when the measurements of the parameter of interest are made.

When the calibrating cover 71B is attached to the baseplate 72, they form together a calibrating housing 7B. The calibrating housing 7B is used when the detecting device 1 needs to be calibrated.

In the following description, if there is no precision of which housing 7A, 7B is described, it means that the feature described relatively to the housing 7A, 7B applies equivalently to the functioning housing 7A and to the calibrating housing 7B. In an equivalent way, if there is no precision of which cover 71A, 71B is described, it means that the feature described relatively to the cover 71A, 71B applies equivalently to the functioning housing 7A and to the calibrating housing 7B.

To attach the calibrating cover 71B or the functioning cover 71A to the baseplate 72, and then respectively form the functioning housing 7A and the calibrating housing 7B, an attaching system 73 may be provided.

More precisely, the attaching system 73 is a reversible system. The attaching system 73 may be a system of clip, of screw, or any suitable system that can be attached and detached easily, i.e. necessitating a minimum of tool and apprehendable by a user who is not an expert.

Hence, for example, the baseplate 72 may be provided with a female and/or a male attaching part on the edge of its lateral surface while the functioning and calibrating covers 71A, 71B are provided with complementary male and/or female attaching part.

When the user needs to detach a cover 71A, 71B, they simply need to unclip the cover 71A, 71B from the baseplate 72. To attach one of the covers to the baseplate 72, the user simply needs to put in regards the complementary female/male parts of the cover 71A, 71B and the baseplate 72 and press the cover and the baseplate together to clip the attaching system.

In another configuration, when the user needs to detach a cover 71A, 71B, they simply need to unscrew the cover 71A, 71B from the baseplate 72. To attach one of the covers to the baseplate 72, the user simply needs to screw back the cover 71A, 71B and the baseplate 72 to lock the attaching system.

Other attaching systems allowing the same functions as described above enter within the scope of the present application.

Regarding the technical problem of providing continuous contact between the medium and the contact surface 92 of the cell 9, elastic elements 110 are provided between the PCB 100 and the inner upper surface 710 of the cover 71. Such elastic elements 110 are able to elastically deform themselves under the effect of a movement of the building block relatively to the housing 7.

A movement of the building block could be induced by an upward force F applied to the surface contact 92 of the cell 9. Such upward force F is represented by the arrow on figure 3. Such upward force would cause an upward displacement of the whole building block relatively to the housing 7.

The elastic elements 110 would absorb the energy generated by the displacement of the building block toward the top of the housing by deforming elastically. The movement of the building block therefore can be compensated, allowing the hollow contact surface 92 of the cell 9 to stay in contact with the medium M.

When the upward force F is applied, the building block will move upwards, towards the inner upper surface 710 of the cover 71, thereby deforming the elastic elements 110 by compressing them between the PCB 100 and the inner upper surface 710 of the cover 71. The elastic elements 110 however will not deform completely beyond their elastic limit but will resist, pushing back the building block towards the baseplate 72.

Hence, in the state where the building block is moving upwards, towards the inner upper surface 710A of the cover 71A, the elastic element 110 is in a compression state, and is pushing downward the building block against the inner lower surface 720 of the baseplate 72.

In the "at rest" state, where the building block is received in the functioning housing 7A without any forces applied on it, for example in a state where the detecting device 1 is not worn by a person P, the elastic elements 110 may be in their rest state in which they are neither elongated nor contracted.

In another embodiment, in the "at rest" state, the elastic elements 110 are in a slightly contracted state, where the slightly contracted state still authorises the elastic element 110 to compress more when the building block is moving towards the inner upper surface 710A of the functioning cover 71A.

The elastic elements 110 may comprise leaf springs. The elastic elements 110 may comprise, alternatively or in complement, spiral springs potentially received in a guiding element to ensure a translation movement according to an only up-down direction, pistons and/or gel.

The elastic elements 110 may also comprise, alternatively or in complement, a layer of elastic material able to compress and elongate as described above.

Any elastic element able to fulfil the above-mentioned functions enter within the scope of the present application.

The detecting device 1 may comprise at least one elastic element 110. Advantageously, the detecting device 1 comprises more than one elastic element 110.

The one or more elastic element(s) 110 may be disposed to compensate for a movement of the single building block. As a non-limitative example, the one or more elastic elements 110 may be disposed symmetrically regarding an axis of measure passing by the centre of and being normal to the hollow contact surface 92.

In an embodiment, the elastic elements 110 are fixed to the PCB 100. In another embodiment, the elastic elements 110 are fixed to the inner upper surface 710A of the functioning cover 71A.

To allow the elastic element 110 to compensate an upward movement of the building block, the height of the elastic element 110 may be slightly inferior to a distance between the building block and the inner upper surface of the building such that, in the functioning housing 7A, the elastic element 110 can be compressed between the building block and the inner upper surface of the functioning cover 71A. Such configuration may allow to facilitate the fabrication of the functioning cover 71A since it may present a flat inner upper surface.

In another embodiment, the elastic element 110 is in regards with a plot 711A emerging out of the inner upper surface of the functioning cover 71A. Such plot 711A reduces the distance between the building block and the inner superior surface of the functioning cover 71A. The distance between the plot 711A and the building block enables the compression of the elastic element 110 between said plot 711A and said building block. Such configuration allows to use a smaller elastic element and to increase the tolerance of fabrication of the functioning cover, thereby reducing the cost and delay of fabrication.

Also, further elastic elements 110 may be provided on the inner lateral sides of the functioning cover 71A to compensate any lateral movement of the building block inside the functioning housing 7A.

As a variant, blocking elements may be provided on the inner lateral sides of the baseplate 72 to block any lateral movement of the building block inside the functioning housing 7A. Such blocking elements may be plots 722 emerging from the inner lateral side of the baseplate 72, where such plots 722 present a same width as a space between the building block and the inner lateral sides of the functioning cover 71A.

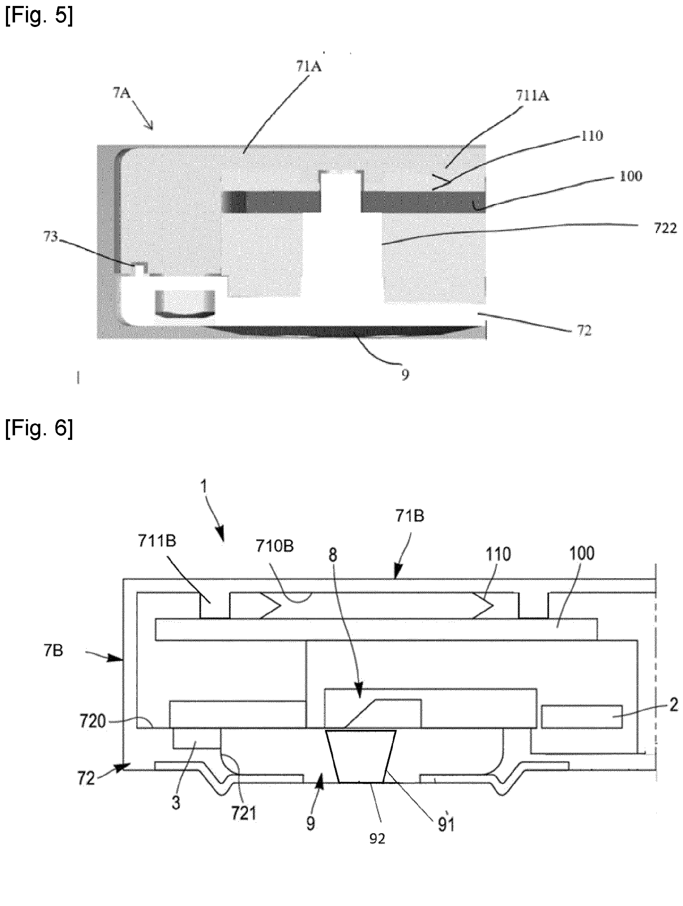

As visible on figure 5, the plots 722 also allow the maintaining element 100 to rest on said plots 722, while authorising an upward movement of said maintaining element 100.

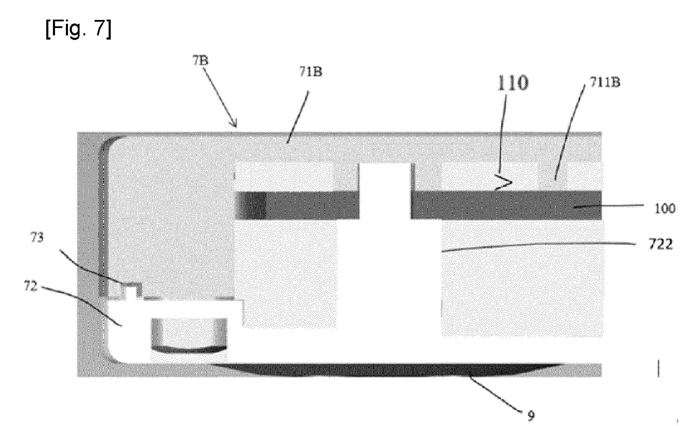

Regarding the calibrating cover 71B more particularly visible on figures 6 and 7, it is intended to be used to form the calibrating housing 7B whenever the detecting device 1 needs to be calibrated. As stated above, the calibration of the detecting device 1 is preferably made while the building block is not moving since the movement of the building block inside the housing would be needed to be known to perform an accurate calibration of the detecting device 1.

To this aim, the inner upper surface of the calibrating cover 71B may comprise at least one plot 711B of a height equal to the distance between the inner upper surface of the calibrating cover 71B and the building block.

Advantageously, such plots 711B are not in regards with the elastic element 110 where the elastic element 110 is provided on top of the building block, such that the plots 711B of the calibrating cover 71B maintains the building block against the inner lower surface of the baseplate 72, and then avoids any upward movement of the building block in the calibrating housing 7B.

Advantageously, the plots 722 of the baseplate 72 may also be able to block any lateral movement of the building block inside the calibrating housing 7B.

As visible on figure 7, the plots 722 of the baseplate 72 also allow the maintaining element 100 to rest on said plots 722, while blocking an upward movement of said maintaining element 100.

While exemplary embodiment of the invention has been described with reference to two main embodiments, it will be understood by those skilled in the art that various changes, omissions and/or additions may be made, and equivalents may be substituted for elements thereof without departing from the scope of the invention as set out in the appended set of claims. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

## Claims

1. Photoacoustic detecting device for measuring a parameter of interest in a medium comprising:
➢ a housing kit comprising:
o a calibrating cover (71B),
o a functioning cover (71A),
o a baseplate (72),
o the calibrating cover (71B) and the functioning cover (71A) being interchangeable with one another, such that,
▪ when the baseplate (72) is attached to the functioning cover (71A), the baseplate (72) and the functioning cover (71A) are configured to form a functioning housing (7A) allowing an analysis of the medium by the photoacoustic detecting device,
▪ when the baseplate (72) is attached to the calibrating cover (71B), the baseplate (72) and the calibrating cover (71B) are configured to form a calibrating housing (7B) allowing a calibration of the photoacoustic detecting device,
➢ a building block freely housed in the housing, said housing being either the functioning housing (7A) or the calibrating housing (7B), said building block comprising:
o a light source (2), configured to emit a light signal,
∘ a photoacoustic cell (9) comprising a contact surface (92) with an opening intended to be in contact with the medium (M), whereby the light signal is able to propagate in the photoacoustic cell (9) and pass through the opening in the contact surface (92) to reach the medium (M), the contact surface of the photoacoustic cell being configured to emerge out of an aperture of the baseplate (72),
o a guiding element (8), configured to direct the light signal toward the photoacoustic cell (9),
o a transducer (3) assembled to the photoacoustic cell (9) and configured to detect a generated signal, where the generated signal is generated in the photoacoustic cell (9) by a photothermic effect in the medium (M) in response to an irradiation of the medium (M) by the light signal,
∘ one or more maintaining elements (100) configured to assemble the light source (2), the transducer (3), the photoacoustic cell (9) and said guide (8) in a single building block,
wherein
➢ in the functioning housing (7A), elastic elements (110) are provided between the functioning cover (71A) and the building block, the functioning cover (71A) being configured to allow a movement of the building block inside the housing, said elastic element (110) being configured to elastically deform under the effect of the movement of the building block relatively to the housing in order to compensate said movement of said building block,
➢ the calibrating cover (71B) comprises blocking elements, said blocking elements being configured to block any movement of the building block relatively to the housing.

2. Device according to claim 1, wherein when the building block is housed in the functioning housing (7A), the building block and an inner upper surface of the functioning cover (71A) are spaced from one another, the elastic element (110) being provided in said space, wherein the building block is configured to move in said space according to an upward-downward movement relatively to the functioning housing (7A) and wherein the elastic element (110) is configured to elastically deform between the functioning cover (71A) and the building block according to the upward-downward movement of said building block in the functioning housing (7A).

3. Device according to any of claims 1 or 2, wherein an upper interior surface of the functioning cover (71A) further comprises a cooperating element cooperating with said elastic element (110).

4. Device according to any of claims 1 to 3, wherein the elastic element (110) is provided on the inner upper interior surface of the functioning cover (71A).

5. Device according to any of claims 1 to 4, wherein the elastic element (110) is provided on the building block.

6. Device according to any of claims 1 to 5, wherein the elastic element (110) is a spring leaf.

7. Device according to any of claims 1 to 6, wherein when the building block is housed in the calibrating housing (7B), the building block and an inner upper surface of the calibrating cover (71B) are spaced from one another, the blocking element of said calibrating cover (71B) comprising a plot of a length equal to said space, said plot thereby being able to maintain the building block against the baseplate (72).

8. Device according to any of claims 1 to 6, wherein the blocking element of the calibrating cover (71B) comprises a height of said calibrating cover (71B) wherein said height of said calibrating cover (71B) is such that a height of the calibrating housing (7B) is equal to a height of the building block, thereby pressing the building block between said calibrating cover (71B) and said baseplate (72).

9. Device according to any of the preceding claims, wherein the baseplate (72) comprises blocking elements on its lateral inner surface, said blocking elements being configured to block any lateral movement of the building block inside the housing.

## Patentansprüche

1. Photoakustische Detektionsvorrichtung zum Messen eines interessierenden Parameters in einem Medium, umfassend:
> einen Gehäusesatz, umfassend:
o eine Kalibrierungsabdeckung (71B),
o eine Funktionsabdeckung (71A),
o eine Grundplatte (72),
o wobei die Kalibrierungsabdeckung (71B) und die Funktionsabdeckung (71A) miteinander austauschbar sind, so dass
▪ wenn die Grundplatte (72) an der Funktionsabdeckung (71A) befestigt ist, die Grundplatte (72) und die Funktionsabdeckung (71A) so konfiguriert sind, dass sie ein Funktionsgehäuse (7A) bilden, das eine Analyse des Mediums durch die photoakustische Detektionsvorrichtung ermöglicht,
▪ wenn die Grundplatte (72) an der Kalibrierungsabdeckung (71B) befestigt ist, die Grundplatte (72) und die Kalibrierungsabdeckung (71B) so konfiguriert sind, dass sie ein Kalibrierungsgehäuse (7B) bilden, das eine Kalibrierung der photoakustischen Detektionsvorrichtung ermöglicht,
> einem frei in dem Gehäuse untergebrachten Baustein, wobei das Gehäuse entweder das Funktionsgehäuse (7A) oder das Kalibrierungsgehäuse (7B) ist, wobei der Baustein umfasst:
o eine Lichtquelle (2), die so konfiguriert ist, dass sie ein Lichtsignal aussendet,
o eine photoakustische Zelle (9) mit einer Kontaktfläche (92) mit einer Öffnung, die dazu bestimmt ist, mit dem Medium (M) in Kontakt zu stehen, wodurch sich das Lichtsignal in der photoakustischen Zelle (9) ausbreiten und durch die Öffnung in der Kontaktfläche (92) hindurchtreten kann, um das Medium (M) zu erreichen, wobei die Kontaktfläche der photoakustischen Zelle so konfiguriert ist, dass sie aus einer Öffnung der Grundplatte (72) herausragt,
o ein Führungselement (8), das so konfiguriert ist, dass es das Lichtsignal zur photoakustischen Zelle (9) lenkt,
o einen Wandler (3), der an der photoakustischen Zelle (9) angebracht und so konfiguriert ist, dass er ein erzeugtes Signal erfasst, wobei das erzeugte Signal in der photoakustischen Zelle (9) durch einen photothermischen Effekt im Medium (M) als Reaktion auf eine Bestrahlung des Mediums (M) durch das Lichtsignal erzeugt wird,
o ein oder mehrere Halteelemente (100), die dazu ausgelegt sind, die Lichtquelle (2), den Wandler (3), die photoakustische Zelle (9) und die Führung (8) in einem einzigen Baustein zusammenzufassen,
wobei
> in dem Funktionsgehäuse (7A) elastische Elemente (110) zwischen der Funktionsabdeckung (71A) und dem Baustein vorgesehen sind, wobei die Funktionsabdeckung (71A) so konfiguriert ist, dass sie eine Bewegung des Bausteins innerhalb des Gehäuses ermöglicht, wobei das elastische Element (110) so konfiguriert ist, dass es sich unter der Wirkung der Bewegung des Bausteins relativ zum Gehäuse elastisch verformt, um die Bewegung des Bausteins auszugleichen,
> die Kalibrierungsabdeckung (71B) Blockierelemente umfasst, wobei die Blockierelemente so konfiguriert sind, dass sie jede Bewegung des Bausteins relativ zum Gehäuse blockieren.

2. Vorrichtung nach Anspruch 1, wobei, wenn der Baustein im Funktionsgehäuse (7A) untergebracht ist, der Baustein und eine innere Oberseite der Funktionsabdeckung (71A) voneinander beabstandet sind, wobei das elastische Element (110) in diesem Raum vorgesehen ist, wobei der Baustein so konfiguriert ist, dass er sich in dem Raum entsprechend einer Aufwärts-Abwärts-Bewegung relativ zum Funktionsgehäuse (7A) bewegt, und wobei das elastische Element (110) so konfiguriert ist, dass es sich entsprechend der Aufwärts-Abwärts-Bewegung des Bausteins im Funktionsgehäuse (7A) zwischen der Funktionsabdeckung (71A) und dem Baustein elastisch verformt.

3. Vorrichtung nach einem der Ansprüche 1 oder 2, wobei eine obere Innenfläche der Funktionsabdeckung (71A) ferner ein mit dem elastischen Element (110) zusammenwirkendes Element umfasst.

4. Vorrichtung nach einem der Ansprüche 1 bis 3, wobei das elastische Element (110) an der inneren oberen Innenfläche der Funktionsabdeckung (71A) vorgesehen ist.

5. Vorrichtung nach einem der Ansprüche 1 bis 4, wobei das elastische Element (110) an dem Baustein vorgesehen ist.

6. Vorrichtung nach einem der Ansprüche 1 bis 5, wobei das elastische Element (110) ein Federblatt ist.

7. Vorrichtung nach einem der Ansprüche 1 bis 6, wobei, wenn der Baustein in dem Kalibriergehäuse (7B) untergebracht ist, der Baustein und eine innere Oberseite der Kalibrierabdeckung (71B) voneinander beabstandet sind, wobei das Sperrelement der Kalibrierabdeckung (71B) einen Abschnitt mit einer Länge umfasst, die gleich dem Abstand ist, wodurch der Abschnitt den Baustein gegen die Grundplatte (72) halten kann.

8. Vorrichtung nach einem der Ansprüche 1 bis 6, wobei das Blockierelement der Kalibrierungsabdeckung (71B) eine Höhe der Kalibrierungsabdeckung (71B) umfasst, wobei die Höhe der Kalibrierungsabdeckung (71B) so bemessen ist, dass eine Höhe des Kalibrierungsgehäuses (7B) gleich einer Höhe des Bausteins ist, wodurch der Baustein zwischen der Kalibrierungsabdeckung (71B) und der Grundplatte (72) gedrückt wird.

9. Vorrichtung nach einem der vorstehenden Ansprüche, wobei die Grundplatte (72) an ihrer seitlichen Innenfläche Sperrelemente aufweist, die so konfiguriert sind, dass sie jede seitliche Bewegung des Bausteins innerhalb des Gehäuses blockieren.

## Revendications

1. Dispositif de détection photoacoustique pour mesurer un paramètre d'intérêt dans un milieu, comprenant :
> un kit de boîtier comprenant :
o un couvercle de calibration (71B),
o un couvercle de fonctionnement (71A),
o une plaque de base (72),
o le couvercle de calibration (71B) et le couvercle de fonctionnement (71A) étant interchangeables l'un avec l'autre, de telle sorte que,
▪ lorsque la plaque de base (72) est fixée au couvercle de fonctionnement (71A), la plaque de base (72) et le couvercle de fonctionnement (71A) sont configurés pour former un boîtier de fonctionnement (7A) permettant une analyse du milieu par le dispositif de détection photoacoustique,
▪ lorsque la plaque de base (72) est fixée au couvercle de calibration (71B), la plaque de base (72) et le couvercle de calibration (71B) sont configurés pour former un boîtier d'étalonnage (7B) permettant un étalonnage du dispositif de détection photoacoustique,
> un bloc fonctionnel logé librement dans le boîtier, ledit boîtier étant soit le boîtier de fonctionnement (7A), soit le boîtier d'étalonnage (7B), ledit bloc fonctionnel comprenant :
o une source lumineuse (2), configurée pour émettre un signal lumineux,
o une cellule photoacoustique (9) comprenant une surface de contact (92) avec une ouverture destinée à être en contact avec le milieu (M), de sorte que le signal lumineux peut se propager dans la cellule photoacoustique (9) et passer à travers l'ouverture dans la surface de contact (92) pour atteindre le milieu (M), la surface de contact de la cellule photoacoustique étant configurée pour émerger d'une ouverture de la plaque de base (72),
o un élément de guidage (8), configuré pour diriger le signal lumineux vers la cellule photoacoustique (9),
o un transducteur (3) assemblé à la cellule photoacoustique (9) et configuré pour détecter un signal généré, où le signal généré est généré dans la cellule photoacoustique (9) par un effet photothermique dans le milieu (M) en réponse à une irradiation du milieu (M) par le signal lumineux,
o un ou plusieurs éléments de maintien (100) configurés pour assembler la source lumineuse (2), le transducteur (3), la cellule photoacoustique (9) et ledit guide (8) en un seul bloc,
dans lequel
> dans le boîtier de fonctionnement (7A), des éléments élastiques (110) sont prévus entre le couvercle de fonctionnement (71A) et le bloc fonctionnel, le couvercle de fonctionnement (71A) étant configuré pour permettre un mouvement du bloc fonctionnel à l'intérieur du boîtier, ledit élément élastique (110) étant configuré pour se déformer élastiquement sous l'effet du mouvement du bloc fonctionnel par rapport au boîtier afin de compenser ledit mouvement dudit bloc fonctionnel,
> le couvercle de calibration (71B) comprend des éléments de blocage, lesdits éléments de blocage étant configurés pour bloquer tout mouvement du bloc fonctionnel par rapport au boîtier.

2. Dispositif selon la revendication 1, dans lequel, lorsque le bloc fonctionnel est logé dans le boîtier de fonctionnement (7A), le bloc fonctionnel et une surface supérieure interne du couvercle de fonctionnement (71A) sont espacés l'un de l'autre, l'élément élastique (110) étant prévu dans ledit espace, dans lequel le bloc fonctionnel est configuré pour se déplacer dans ledit espace selon un mouvement vers le haut et vers le bas par rapport au boîtier de fonctionnement (7A) et dans lequel l'élément élastique (110) est configuré pour se déformer élastiquement entre le couvercle de fonctionnement (71A) et le bloc fonctionnel en fonction du mouvement vers le haut et vers le bas dudit bloc fonctionnel dans le boîtier de fonctionnement (7A).

3. Dispositif selon l'une quelconque des revendications 1 ou 2, dans lequel une surface intérieure supérieure du couvercle de fonctionnement (71A) comprend en outre un élément coopérant avec ledit élément élastique (110).

4. Dispositif selon l'une quelconque des revendications 1 à 3, dans lequel l'élément élastique (110) est prévu sur la surface intérieure supérieure du couvercle de fonctionnement (71A).

5. Dispositif selon l'une quelconque des revendications 1 à 4, dans lequel l'élément élastique (110) est prévu sur le bloc fonctionnel.

6. Dispositif selon l'une quelconque des revendications 1 à 5, dans lequel l'élément élastique (110) est un ressort en lame.

7. Dispositif selon l'une quelconque des revendications 1 à 6, dans lequel, lorsque le bloc fonctionnel est logé dans le boîtier de calibration (7B), le bloc fonctionnel et une surface supérieure interne du couvercle de calibration (71B) sont espacés l'un de l'autre, l'élément de blocage dudit couvercle de calibration (71B) comprenant une partie d'une longueur égale audit espace, ladite partie pouvant ainsi maintenir le bloc fonctionnel contre la plaque de base (72).

8. Dispositif selon l'une quelconque des revendications 1 à 6, dans lequel l'élément de blocage du couvercle de calibration (71B) comprend une hauteur dudit couvercle de calibration (71B), ladite hauteur dudit couvercle de calibration (71B) étant telle qu'une hauteur du boîtier de calibration (7B) est égale à une hauteur du bloc fonctionnel, pressant ainsi le bloc fonctionnel entre ledit couvercle de calibration (71B) et ladite plaque de base (72).

9. Dispositif selon l'une quelconque des revendications précédentes, dans lequel la plaque de base (72) comprend des éléments de blocage sur sa surface latérale intérieure, lesdits éléments de blocage étant configurés pour bloquer tout mouvement latéral du bloc fonctionnel à l'intérieur du boîtier.
